# EUROPÄISCHE PATENTSCHRIFT

(11) **EP 1 511 521 B1**
(45) Veröffentlichungstag und Bekanntmachung des Hinweises auf die Patenterteilung: **09.04.2008**
(21) Anmeldenummer: 03735389.3
(22) Anmeldetag: 14.05.2003
(51) Int. Cl.: A61L 24/00, A61L 27/12, A61L 27/54, A61L 27/58, A61L 31/12, A61L 31/16, A61L 31/14, A61K 6/033, C04B 28/34

(54) **STRONTIUM-APATIT-ZEMENT-ZUBEREITUNGEN UND DEREN VERWENDUNGEN**
STRONTIUM-APATITE-CEMENT-PREPARATIONS AND THE USE THEREOF
PREPARATIONS DE CIMENT D'APATITE DE STRONTIUM ET LEURS UTILISATIONS

(30) Priorität: 07.06.2002 DE 10225420
(43) Veröffentlichungstag der Anmeldung: 09.03.2005
(73) Patentinhaber: Kyphon Inc., Sunnyvale, CA 94089 (US)
(72) Erfinder: WENZ, Robert, 61206 Wöllstadt (DE)
(74) Vertreter: Hofer, Dorothea
(86) Internationale Anmeldenummer: PCT/EP2003/005059
(87) Internationale Veröffentlichungsnummer: WO 2003/103734

(56) Entgegenhaltungen:
- EP-A- 0 473 048
- EP-A- 0 511 868
- EP-A- 0 543 765
- EP-A- 1 002 513
- WO-A-01/49327
- WO-A-02/02478
- US-B1- 6 338 810

## Beschreibung

Die Erfindung betrifft Calcium-Strontium-Hydroxyphosphat-(Strontium-Apatit-) Zementzubereitungen, die Calcium und Strontium enthalten, und deren Verwendungen. Die Erfindung betrifft ferner Strontium-Apatit-Zemente, die aus diesen Zementzubereitungen gebildet werden, und das zu ihrer Herstellung angewandte Verfahren. Das Strontium-Apatit eignet sich gut für medizinische Zwecke, insbesondere als Knochenersatzmaterials, mit spezieller Eignung zur Auffüllung von Osteoporose bedingten Knochendefekten.

Das menschliche und tierische Hartgewebe besteht im wesentlichen aus Hydroxylapatit, wobei meist kein stöchiometrischer Hydroxylapatit vorliegt, sondern eine Apatitstruktur, in welche Na, K, Mg und Strontium Salze mit eingebaut sind.
In das Hartgewebe ist überdies noch Carbonat eingebaut, welches im Austausch gegen Phosphatgruppen in die Apatitstruktur eingebaut wird.

Physiologisch vorkommender Apatit ist nanokristallin, was sich im Röntgendiffraktogramm in Form einer Linienverbreiterung darstellt, die keine exakte Zuordnung der Apatitstrukturen zulässt, da es sich vielmehr um die Superposition einzelner Peaks handelt.

Calciumphosphate sind biokompatibel und osteokonduktiv, was bedeutet, dass sich neugebildetes Knochengewebe direkt auf diesen abscheidet. Sie sind überdies resorbierbar, weil sie als körpereigen erkannt werden und im Rahmen des natürlichen Knochenmetabolismus und Umbaus von spezifischen knochenresorbierenden Zellen, den Osteoklasten, abgebaut werden können. Während solcher Umbauvorgänge können Calciumphosphate abgebaut und durch körpereigenen Knochen ersetzt werden.

Seit ca. 1970 gibt es Calciumphosphat-Keramiken auf dem Markt, die vorwiegend in Form von vorgefertigten Formkörpern oder als Granulate in den menschlichen und tierischen Körper eingebaut werden. Diese Materialien haben sich im klinischen Einsatz bewährt, sie können jedoch nur selten kraftschlüssig in Defekte, die meist irregulär sind, eingebaut werden. Durch fehlenden kraftschlüssigen Einbau kommt es aber oftmals zum Ausschwemmen der Granulate oder zum Einwachsen von Bindegewebe in den Defekt. Dies führt dann zum Versagen der Augmentation.

Calciumphosphat-Keramiken werden vorwiegend aus Hydroxylapatit hergestellt, wobei diese Keramiken nicht resorbierbar sind oder aus biphasischen Calciumphosphat-Keramiken, die aus variablen Anteilen von β-Tricalciumphosphat (β-TCP) und Hydroxylapatit bestehen und wegen der Resorbierbarkeit des β-Tricalciumphosphates zumindest teilweise, entsprechend dem Masseanteil, resorbiert werden können.

Calciumphosphat-Zemente wurden erstmals seit 1985 in der Literatur erwähnt. Sie haben Vorteile gegenüber den Keramiken dadurch, dass sie kraftschlüssig in den Körper eingebracht werden können (W.E. Brown and L.C. Chow, "A new calcium phosphate, watersetting cement", Chem. Res. Prog. 1986, 352-379; US 4,612,053; US 5,149,368; US 4,518,430; WO 96/14265; EP 0 835 668 A1).
Diese Zemente zeichnen sich durch ein Ca/P Verhältnis ≥ 1,5 aus.
Durch Carbonat-Zugabe kann dieses Verhältnis noch vergrößert werden. Über die Resorbierbarkeit dieser Materialien gibt es widersprüchliche Berichte, weil solche Zemente, wenn das Reaktionsendprodukt Hydroxylapatit ist, nicht resorbiert werden, oder, falls es sich als Reaktionsendprodukt um Calciumdefizienten-Hydroxylapatit (CDHA) handelt, dieser durch Osteoklasten resorbiert und von Osteoblasten durch neuen Knochen ersetzt werden kann. Die Resorptionsgeschwindigkeit ist dann nicht vorhersehbar, weil die Resorption dann abhängig ist von der zellulären Aktivität des Empfängers, der lokalen Durchblutungsrate und dem Implantationsort.

Solche Zemente sind bereits erfolgreich auf dem Markt eingeführt (BoneSource, Norian SRS, Biobon, Calcibon). Ein Hauptkritikpunkt von Seiten der Anwender ist allerdings die nicht vorhersehbare Resorbierbarkeit. Der Markt verlangt nach einem Produkt, welches eine hohe mechanische Stabilität garantiert, aber nach einer bestimmten Zeit vollständig resorbiert und durch körpereigenen Knochen ersetzt sein sollte.

Viele Hersteller fügen aus diesem Grund ihren Knochenersatzmaterialien lösliche Mineralien wie CaHPO₄, CaSO₄, CaCO₃ oder β-TCP zu, um über die passive Löslichkeit die Resorptionsgeschwindigkeit zu erhöhen. Dies löst das Problem jedoch nur teilweise, weil die Hauptkomponente immer noch schwer oder nicht resorbierbar bleibt.
Die aufgrund zellulärer Phänomene gesteuerte Zementresorption folgt den Regeln des Wolff'schen Gesetzes. Das Wolff'sche Gesetz beschreibt die ständigen Knochenumbaumodalitäten, und seine Kernaussage ist die, dass Knochen nur dort verbleibt, wo er aus biomechanischer Sicht auch benötigt wird. Aus dieser Aussage folgt, dass sich die Druckfestigkeit eines künstlichen Knochenersatzwerkstoffes an der des trabekulären Knochens orientieren sollte.
Das heißt, eine höhere Druckfestigkeit > 40 MPa ist gar nicht wünschenswert, weil sonst ein gewisses "Stress-shielding" durch den Zement erzeugt wird, welches, bedingt durch die höhere Festigkeit des Zementes, die Knochenstruktur des angrenzenden Implantatlagers auflockert. Damit wird der Ort der geringsten biomechanischen Festigkeit vom Zement in das äußere Implantatlager verschoben, welches nicht wünschenswert ist.

Die Hauptverwendung von Knochenersatzwerkstoffen liegt in der Auffüllung von metaphysären Knochendefekten und von Wirbelkörpern. Diese Defekte entstehen hauptsächlich im Verlauf der Osteoporose. Die Osteoporose ist eine systemische Erkrankung des Gesamtorganismus, die sich im wesentlichen durch eine Imbalance des Knochenstoffwechsels ausdrückt. Dabei kehren sich die anabolen und katabolen Knochenumbauvorgänge um, und es wird mehr Knochen durch osteoklastäre Aktivität abgebaut als durch Osteoblastenaktivität angebaut wird. Diesem Ungleichgewicht der Knochenabbaurate zur Knochenanbaurate wird versucht mit verschiedenen systemisch wirksamen Substanzen entgegenzutreten. Dazu zählen unter anderem die Bisphosphonate und Hormonpräparate, die jedoch den Gesamtorganismus belasten. Wünschenswert wäre in dieser Hinsicht ein Knochenersatzmaterial, welches sich dadurch auszeichnet, dass es nicht nur ein reiner Knochenersatzstoff bzw. Füllstoff darstellt, sondern dass dieses Material auf die es umgebenden Knochenzellen derart wirkt, dass es die metabolischen Vorgänge gewissermaßen umkehrt, so dass eine überschießende osteoklastäre Aktivität durch das Knochenersatzmaterial selbst attenuiert wird und die osteoblastäre (Knochen anbauende) Aktivität stimuliert wird. Durch die Vorgabe eines solchen Entwicklungszieles wird es vermieden, dass infolge osteoporotischer Aktivitäten ein einmal eingebrachtes Knochenersatzmaterial durch die gesteigerte Osteoklastenaktivität schnell abgebaut wird, ohne dass gleichzeitig, wegen der Osteoporose bedingten attenuierten Osteoblastentätigkeit, neuer Knochen angebaut werden kann.

Diese Probleme werden beim Betrachten des gegenwärtigen Standes der Technik nicht gelöst. In der WO 02/02478 A1 wird zwar ein Calciumphosphat-Zement offenbart, welcher Strontium-Ionen in Form von SrCO₃ enthält, hierbei wird das Strontiumcarbonat jedoch nur zur Beeinflussung der expansiven Eigenschaften des in seiner Hauptkomponente aus Magnesium-ammonium-phosphat bestehenden Zementes verwendet. Dieses Strontiumcarbonat wird zudem, wegen seiner potenziellen Löslichkeit schnell aus dem Zement herausgelöst, so dass von diesem kein protrahierter Effekt ausgehen kann und somit der Knochenmetabolismus nicht beeinflusst werden kann.

Aufgabe der Erfindung ist es, ein Material bereit zu stellen, welches sich als Knochenersatzmaterial, insbesondere für den osteoporotischen Knochen, besonders gut eignet.

Diese Aufgabe wird besonders gelöst durch die Zement-Zubereitung gemäß Anspruch 1 oder Anspruch 11, durch die Verwendungen dieser Zement-Zubereitungen gemäß den Ansprüchen 21 und 22, durch das Verfahren zur Herstellung eines Strontium-Apatit-Zements gemäß Anspruch 23 sowie durch das aus diesen Zement-Zubereitungen gebildete Strontium-Apatit gemäß Anspruch 26.
Bevorzugte Ausführungsformen sind in den Unteransprüchen zu den oben genannten Ansprüchen wiedergegeben.

Die erfindungsgemäßen Zement-Zubereitungen zeichnen sich durch die Gegenwart von Calcium- und Strontium-Ionen aus. Diese Calcium- und Strontium-Ionen können in Form ihrer Phosphate oder Hydrogenphosphate, gegebenenfalls zusätzlich auch ihrer Carbonate, in die Zubereitungen eingebracht werden. Magnesium-Ionen können in unvermeidbaren Spuren vorliegen.

Aufgrund seiner Zusammensetzung besitzen die erfindungsgemäßen Zement-Zubereitungen und damit das daraus gebildete, ausgehärtete Strontium-Apatit die Fähigkeit, protrahiert Strontiumionen freizusetzen. Dadurch besteht die Möglichkeit, dass der Knochenstoffwechsel im osteoporotischen Knochen positiv beeinflusst wird, insbesondere im osteoporotischen Knochen eine anabole Aktivität im Knochenstoffwechsel erzeugt werden kann. Es wird dadurch, insbesondere im osteoporotischen Knochen, eine ständige Stimulation der Osteoblastentätigkeit bewirkt wird bei gleichzeitiger Inhibition der bei der Osteoporose verstärkten Osteoklastenaktivität.

Mit der vorliegenden Erfindung wird ferner ein Zementsystem zur Verfügung gestellt, mit welchem Knochendefekte formschlüssig ausgefüllt werden können, wobei die Zement-Zubereitungen nicht nur bei Raumtemperatur, sondern auch bei Körpertemperatur aushärtbar und für den Anwender ausreichend lange verarbeitbar sind. Nach ihrer Aushärtung weisen die erfindungsgemäßen Zement-Zubereitungen im menschlichen oder tierischen Körper eine passend hohe Druckfestigkeit auf. Das aus der Zement-Zubereitung gebildete Calcium-Strontium-Hydroxyphosphat (Strontium-Apatit) ist nanokristallin und erreicht seine maximale Festigkeit in einer Zeit von wenigen Stunden bis wenigen Tagen.

Die Zementzubereitungen der Erfindung sind, je nach biologischer Situation, biologisch abbaubar bzw. können im Körper resorbiert werden. Dabei zeichnet sich das erfindungsgemäße Material durch eine gute Kohäsionsfähigkeit bei Kontakt mit Körperflüssigkeiten aus. Das erfindungsgemäße Material weist eine höhere Wasserlöslichkeit auf als Calciumdefizienter-Hydroxylapatit, was sich günstig auf den Ersatz durch körpereigenen Knochen auswirkt.

Somit wird gemäß der vorliegenden Erfindung ein Material bereitgestellt, welches aufgrund seiner Zusammensetzung nicht nur Knochendefekte schließen, sondern auch eine anabole Aktivität im Knochen erzeugen und damit aktiv zum Knochenaufbau beitragen kann.

Die Erfindung und bevorzugte Ausführungsformen davon werden nachfolgend näher erläutert.

Die im Anspruch 1 definierte Zement-Zubereitung gemäß der Erfindung weist in der Pulvermischung ein molares Ca/P- und Sr/P-Verhältnis in den Bereichen 1,00 < Ca/P ≤ 1,50 und 0 < Sr/P < 1,50 auf. Das Sr/P-Verhältnis beträgt vorzugsweise mindestens 0,2 und weiter bevorzugt mindestens 0,5.

Die im Anspruch 11 definierte Zement-Zubereitung gemäß der Erfindung ist durch die chemische Zusammensetzung der Ausgangskomponenten zur Bildung des Strontium-Apatit-Zements bestimmt und enthält in der Pulvermischung als Mindestbestandteile neben Ca₃(PO₄)₂ (TCP), das als α- und/oder β-TCP vorliegen kann, SrHPO₄ und/oder Sr₃(PO₄)₂ und gegebenenfalls zusätzlich SrCO₃.

Die nachfolgende Beschreibung bezieht sich auf beide Gegenstände der Erfindung.

Das Alkalisalz oder das Ammoniumsalz der Orthophosphorsäure kann als Ausgangsmaterial der Zubereitung getrennt von Pulvermischung und Wasser und/oder wässriger Lösung vorliegen. Bevorzugt liegt das Salz in Form der wässrigen Lösung vor, die als Anmischflüssigkeit zum Bilden des Zements mit der trockenen Pulvermischung vermischt wird. Zum Steuern der Reaktionsgeschwindigkeit kann zusätzliches Alkalisalz oder Ammoniumsalz in trockener Form in der Pulvermischung vorliegen. Hierzu enthält die Pulvermischung vorzugsweise zusätzlich NaH₂PO₄ und/oder Na₂HPO₄, KH₂PO₄ und/oder K₂HPO₄ oder Kombinationen der genannten Na- und K-Salze der Orthophosphorsäure.

Als Alkalisalz für die wässrige Lösung der Anmischflüssigkeit ist insbesondere ein Na- und/oder ein K-Salz der Orthophosphorsäure geeignet, speziell der primären oder sekundären Salze und insbesondere deren Kombination. Bevorzugt wird das Alkalisalz der Orthophosphorsäure aus einer Gruppe ausgewählt, die aus folgenden Bestandteilen besteht: ein primäres Kaliumsalz (KHₐPO₄), ein sekundäres Kaliumsalz (K₂HPO₄) der Orthophosphorsäure und eine Mischung davon, und ein primäres Natriumsalz (NaH₂PO₄), ein sekundäres Natriumsalz (Na₂HPO₄) der Orthophosphorsäure und eine Mischung davon, und Kombinationen der genannten Kalium- und Natriumsalze. Als Ammoniumsalz für die wässrige Lösung der Anmischflüssigkeit ist (NH₄)₂HPO₄ besonders geeignet.

Damit Strontium-Ionen günstig in die Strontium-Apatit-Struktur eingebaut werden kann, liegt das Strontium in der Pulvermischung vorteilhaft als Strontiumphosphat (Sr₃(PO₄)₂ oder Strontiumhydrogenphosphat (SrHPO₄) oder einer Mischung davon vor. Die Menge an SrHPO₄ und/oder Sr₃(PO₄)₂ in der Pulvermischung beträt vorzugsweise mehr als 10 Gew.-% bis 60 Gew.-%. Weiter bevorzugt sind mehr als 15 Gew.-%, insbesondere mehr als 20 Gew.-% davon enthalten.
Das wahlweise zusätzlich in die Pulvermischung eingebrachte SrCO₃ kann in einer Menge von zum Beispiel 0,01 bis 10 Gew.-% vorliegen.

Die Pulvermischung kann zusätzlich je nach Wunsch weitere geeignete Substanzen enthalten, z.B. Metall-Carbonate, Ca-, Mg- , Sr-, Na-, K-Sulfate, Ca-, Na-, K-Phophate, Ca-, Na-, K-Hydrogenphosphate sowie deren Oxide und/oder -Hydroxyde.

Zur Herstellung des Strontium-Apatit-Zements wird die oben beschriebene Pulverkomponente mit der oben beschriebenen wässrigen Komponente (der Anmischlösung) gemischt und diese Mischung anschließend aushärten gelassen, so dass Strontium-Apatit als Reaktionsendprodukt gebildet wird. Dabei kann die Pulvermischung sowohl mit alkalischen Lösungen, die (NH₄)₂HPO₄, K₂HPO₄ und/oder Na₂HPO₄ enthalten, als auch mit sauren Lösungen, die NaH₂PO₄ und/oder KH₂PO₄ enthalten, oder durch geeignete Mischlösungen der genannten primären und sekundären Orthophosphate zum Aushärten gebracht werden. Die wässrige Lösung weist vorzugsweise einen pH-Wert im Bereich von 5 bis 12 auf.

Nach dem Mischen wird üblicherweise eine Paste gebildet. Diese kann in eine Form eingefüllt werden, wodurch nach der Aushärtung der Paste, der Form als Matrix folgend, definierte Formkörper hergestellt werden können. Die Viskosität und/oder die Konsistenz der Mischung kann dabei derart eingestellt werden, dass diese nicht nur als Paste mit Werkzeugen in Defekte eingebracht, sondern auch injiziert werden kann.

Die Zement-Zubereitung bzw. das Strontium-Apatit-Zement gemäß der Erfindung kann für medizinische Zwecke eingesetzt werden und ist als Knochenersatzstoff, als Knochenfüllstoff, als Knochenzement, als Knochenklebstoff und vor allem als therapeutisches Mittel zur Behandlung der Osteoporose besonders geeignet.

Die Zement-Zubereitung bzw. das Strontium-Apatit-Zement gemäß der Erfindung ist außerdem als Trägermaterial für Wirkstoffe biologischer oder pharmazeutischer Herkunft geeignet. Hierzu enthält die Zubereitung in der Pulver- und/oder der wässrigen Flüssigkeitskomponente zusätzlich eine pharmakologisch und/oder biologisch wirksame Substanz, etwa ein Antibiotikum, ein Zytostatikum, ein Analgetikum, ein Desinfektionsmittel, ein Wachstumsfaktor, ein Protein oder ein Biopolymer bzw. Kombinationen der genannten Wirksubstanzen. Besonders geeignet ist der Einsatz eines Wirkstoffs aus der Gruppe Gentamicin bzw. Tobramycin, Clindamycin und Vancomycin, ein Wirkstoff der TGF-ß-Reihe oder aus der Reihe der BMPs bzw. Kombinationen der genannten Wirkstoffe.

Eine weitere bevorzugte Ausführungsform besteht darin, dass die Zubereitung in der Pulverkomponente zusätzlich eine Substanz in Form von granulären Partikeln enthält, die sich in der wässrigen Flüssigkeitskomponente löst, wie z.B. Salze, Zucker, synthetische, hydrolytisch abbaubare Polymere. Diese granulären Partikel, die z.B. in einer Korngröße von 10 bis 300 µm eingebracht werden, generieren dann nach dem Mischen und während des Aushärtungsprozess ein Porensystem, wodurch die Oberfläche vergrößert und die Resorptionsleistung beschleunigt wird.

Die Erfindung wird nachfolgend anhand von nicht einschränkenden Beispielen näher erläutert.

### Beispiele:

In den Beispielen werden folgende Abkürzungen verwendet:
- P =: Pulvermischung
- L =: Flüssigkeit
- L/P =: Flüssigkeits / Pulver Verhältnis in ml / g
- tᵢ =: initiale Aushärtezeit (nach ASTM C266-89)
- t_{f} =: finale Aushärtezeit (nach ASTM C266-89)
- Cₛ(xh/yd) =: Kompressionsfestigkeit in MPa nach x Stunden/y Tage Lagerung in 37°C warmer 0,9%-iger Kochsalzlösung
- MPa =: Mega Pascal

Herstellung von Strontium-Apatit-Zement-Zubereitungen und daraus gebildete Zemente:
Wie in den nachfolgenden Beispielen 1 bis 7 angegeben wurden nach dem Einwiegen aller Bestandteile die Pulverkomponenten P in einer Kugelmühle miteinander homogen vermahlen und anschließend mit einer wässrigen Lösung L in dem angegebenen Verhältnis vermischt. Nach Ablauf einer bestimmten Aushärtungsdauer wurde jeweils die Kompressionsfestigkeit bestimmt.

### Beispiel 1:

P = 65g Ca₃(PO₄)₂ + 16g Sr₃(PO₄)₂
L = 3,5 M (NH₄)₂HPO₄
L/P = 0,40
Cₛ(48h) = 30 MPa
Cₛ(10d) = 43,8 MPa

### Beispiel 2:

P = 65g Ca₃(PO₄)₂ + 16g Sr₃(PO₄)₂
L = 4% Na₂HPO₄
L/P = 0,35
ti = 13'30" (13 Minuten und 30 Sekunden)

### Beispiel 3:

P = 65g Ca₃(PO₄) + 16g Sr₃(PO₄)₂ + 3g SrCO₃
L = 3,5 M (NH₄)₂HPO₄
L/P = 0,40
Cₛ((10d) = 46,4 Mpa

### Beispiel 4:

P = 60g Ca₃(PO₄)₂ + 10g Sr₃(PO₄)₂ + 10g SrHPO₄ + 3g SrCO₃
L = 3M K₂HPO₄/1M KH₂PO₄
L/P = 0,40
Cₛ(2h) =3,8 Mpa
Cₛ(18h) = 26, 4 Mpa

### Beispiel 5:

P = 65g Ca₃(PO₄)₂ + 16g SrHPO₄ + 3g SrCO₃
L = 3M K₂HPO₄/1M KH₂PO₄
L/P = 0,30
Cₛ(5h) = 18,4 Mpa

### Beispiel 6:

P = 65g Ca₃(PO₄)₂ + 12g Sr₃(PO₄)₂ + 14g SrHPO₄ + 3g SrCO₃
L = 3,2M (NH₄)₂HPO₄
L/P = 0,35
Cₛ(5h) = 13,0 Mpa

### Beispiel 7:

P = 30g Ca₃(PO₄)₂ + 10g Sr₃(PO₄)₂ + 10g SrHPO₄ + 5g SrCO₃ + 10g K₂HPO₄
L = 3M K₂HP0₄/1M KH₂PO₄
L/P = 0,22
Cₛ(72h) = 40 Mpa

## Patentansprüche

1. Härtbare Calcium-Strontium-Hydroxyphosphat-Zement-Zubereitung, umfassend:
- eine Pulvermischung mit molaren Mengen der Komponenten Calcium (Ca), Strontium (Sr) und Orthophosphat (P) in der Mischung in den Bereichen 1,00 < Ca/P ≤ 1,50 und 0 < Sr/P < 1,50
- ein Alkalisalz oder ein Ammoniumsalz der Orthophosphorsäure,
und
- Wasser und/oder eine wässrige Lösung,
wobei die zubereitung Magnesium-Ionen nur in unvermeidbaren Spüren enthalten kann.

2. Zubereitung nach Anspruch 1, wobei das Alkalisalz der Orthophosphorsäure ein Natrium- (Na-) und/oder Kalium- (K-) Salz ist.

3. Zubereitung nach Anspruch 1 oder 2, wobei das Alkalisalz der Orthophosphorsäure aus einer Gruppe ausgewählt ist, die aus folgenden Bestandteilen besteht:
ein primäres Kaliumsalz, ein sekundäres Kaliumsalz der Orthophosphorsäure und eine Mischung davon,
ein primäres Natriumsalz, ein sekundäres Natriumsalz der Orthophosphorsäure und eine Mischung davon,
und Kombinationen der genannten Kalium- und Natriumsalze.

4. Zubereitung nach einem der vorangehenden Ansprüche, wobei das Alkali- oder Ammoniumsalz der Orthophosphorsäure in der wässrigen Lösung vorliegt.

5. Zubereitung nach einem der vorangehenden Ansprüche, wobei die wässrige Lösung einen pH-Wert im Bereich von 7 bis 12 aufweist.

6. Zubereitung nach einem der vorangehenden Ansprüche, wobei das Strontium in der Pulvermischung als Strontiumphosphat oder Strontiumhydrogenphosphat oder einer Mischung davon vorliegt.

7. Zubereitung nach Anspruch 6, wobei das Strontiumphosphat, das Strontiumhydrogenphosphat oder die Mischung davon in der Pulvermischung bei einer Menge von mehr als 10 Gew.-% bis 60 Gew.-% vorliegt.

8. Zubereitung nach Anspruch 6, wobei die Pulvermischung zusätzlich Strontiumcarbonat enthält.

9. Zubereitung nach einem der vorangehenden Ansprüche, wobei die Pulvermischung folgende Bestandteile enthält:
- Ca₃(PO₄)₂ (TCP),
- SrHPO₄ und/oder Sr3(PO₄)₂
- gegebenenfalls zusätzlich SrCO₃.

10. Zubereitung nach Anspruch 9, wobei die Pulvermischung zusätzlich NaH₂PO₄ und/oder Na₂HPO₄, KH₂PO₄ und/oder K₂HPO₄ oder Kombinationen der genannten Na- und K-Salze der Orthophosphorsäure enthält.

11. Strontium-Apatit-Zement-Zubereitung mit einer Pulvermischungs- und einer wässrigen Anmischlösungs-Komponente,
wobei die Pulvermischungs-Komponente folgende Bestandteile enthält:
- Ca₃(PO₄)₂ (TCP),
- SrHPO₄ und/oder Sr₃(PO₄)₂,
- gegebenenfalls zusätzlich SrCO₃,
und wobei die Anmischlösungs-Komponente eine wässrige Lösung eines Alkalisalzes oder eines Ammoniumsalzes der Orthophosphorsäure enthält, wobei die Zubereitung Magnesium-Ionen nur in unvermeidbaren Spuren enthalten kann.

12. Strontium-Apatit-Zement-Zubereitung gemäß Anspruch 11, wobei die Anmischlösungs-Komponente eine wässrige Lösung von (NH₄)₂HPO₄ oder eine wässrige Lösung eines primären und/oder sekundären Na- und/oder K-Salzes der Orthophosporsäure enthält.

13. Strontium-Apatit-Zement-Zubereitung gemäß Anspruch 11, wobei die Anmischlösungs-Komponente eine wässrige Lösung von primärem Salz NaH₂PO₄ und/oder KH₂PO₄, eine wässrige Lösung von sekundärem Salz K₂HPO₄ und/oder Na₂HPO₄, oder eine wässrige Lösung einer Mischung aus primärem Salz KH₂PO₄ und/oder NaH₂PO₄ mit sekundärem Salz K₂HPO₄ und/oder Na₂HPO₄ enthält.

14. Strontium-Apatit-Zement-Zubereitung gemäß Anspruch 11, wobei die Pulvermischung zusätzlich NaH₂PO₄ und/oder Na₂HPO₄, KH₂PO₄ und/oder K₂HPO₄ oder Kombinationen der genannten Na- und K-Salze der Orthophosphorsäure enthält.

15. Strontium-Apatit-Zement-Zubereitung gemäß einem der Ansprüche 11 bis 14, wobei in der Pulvermischungs-Komponente SrHPO₄ und/oder Sr₃(PO₄)₂ in einer Menge von mehr als 10 Gew.-% bis 60 Gew.-% vorliegt.

16. Strontium-Apatit-Zement-Zubereitung gemäß einem der Ansprüche 11 bis 15, wobei in der Pulvermischungs-Komponente SrCO₃ in einer Menge von 0,01 bis 10 Gew.-% vorliegt.

17. Zubereitung gemäß einem der Ansprüche 1 bis 16, wobei die Pulver- und/oder die wässrige Flüssigkeitskomponente zusätzlich eine pharmakologisch und/oder biologisch wirksame Substanz enthält.

18. Zubereitung gemäß Anspruch 17, wobei die pharmakologisch und/oder biologisch wirksame Substanz ein Antibiotikum, ein Zytostatikum, ein Analgetikum, ein Desinfektionsmittel, ein Wachstumsfaktor, ein Protein und/oder ein Biopolymer ist.

19. Zubereitung gemäß Anspruch 18, wobei die pharmakologisch und/oder biologisch wirksame Substanz eine solche ist, die aus der folgenden Gruppe ausgewählt ist: Gentamicin bzw. Tobramycin, Clindamycin, Vancomycin, eine Substanz der TGF-β-Reihe und eine Substanz aus der Reihe der BMPs.

20. Zubereitung gemäß einem der Ansprüche 1 bis 19, wobei die Pulverkomponente zusätzlich eine Substanz in Form von granulären Partikeln enthält, die sich in der wässrigen Flüssigkeitskomponente löst.

21. Verwendung einer Zement-Zubereitung gemäß einem der Ansprüche 1 bis 20 zur Herstellung eines Mittels für medizinische Zwecke.

22. Verwendung einer Zubereitung gemäß einem der Ansprüche 1 bis 20 zur Herstellung eines Knochenersatzstoffs, eines Knochenfüllstoffs, eines Knochenzements oder eines Knochenklebstoffs oder eines therapeutischen Mittels zur Behandlung der Osteoporose.

23. Verfahren zur Herstellung eines Strontium-Apatit-Zements mit den Schritten:
- Mischen einer Pulverkomponente und einer wässrigen Komponente, wie sie in einem der Ansprüche 1 - 19 angegeben sind, und
- Aushärten lassen der Mischung unter Bildung von Strontium-Apatit als Reaktionsendprodukt.

24. Verfahren nach Anspruch 23, wobei eine nach dem Mischen gebildete Paste in eine Form eingefüllt wird, wodurch nach der Aushärtung der Paste der Form als Matrix folgend definierte Formkörper hergestellt werden.

25. Verfahren nach Anspruch 23, wobei nach dem Mischen und vor dem Aushärten der Mischung eine injizierbare Flüssigkeit gebildet wird.

26. Strontium-Apatit, erhältlich durch ein Verfahren zur Herstellung eines Strontium-Apatit-Zements gemäß Anspruch 23.

## Claims

1. A hardenable calcium strontium hydroxyphosphate cement preparation comprising:
- a powder mixture with molar amounts of the components calcium (Ca), strontium (Sr) and phosphate (P) in the mixture in the ranges 1.00 < Ca/P ≤ 1.50, and 0 < Sr/P < 1.50,
- an alkali salt or an ammonium salt of orthophosphoric acid, and
- water and/or an aqueous solution,
wherein the preparation may contain magnesium ions only in unavoidable traces.

2. The preparation according to claim 1, wherein the alkali salt of the orthophosphoric acid is a sodium- (Na-) and/or potassium- (K-) salt.

3. The preparation according to claim 1 or 2, wherein the alkali salt of the orthophosphoric acid is selected from the group consisting of the following components:
a primary potassium salt, a secondary potassium salt of the orthophosphoric acid and a mixture thereof,
a primary sodium salt, a secondary sodium salt of the orthophosphoric acid and a mixture thereof,
and combinations of said potassium and sodium salts.

4. The preparation according to one of the preceding claims, wherein the alkali or ammonium salt of the orthophosphoric acid is present in the aqueous solution.

5. The preparation according to one of the preceding claims, wherein the aqueous solution has a pH value in the range of 7 to 12.

6. The preparation according to one of the preceding claims, wherein the strontium is present in a powder mixture as strontium phosphate or strontium hydrogenphosphate, or a mixture thereof.

7. The preparation according to claim 6, wherein the strontium phosphate, the strontium hydrogenphosphate, or the mixture thereof is present in the powder mixture in an amount of more than 10 wt.-% to 60 wt.-%.

8. The preparation according to claim 6, wherein the powder mixture additionally contains strontium carbonate.

9. The preparation according to one of the preceding claims, wherein the powder mixture contains the following components:
- Ca₃(PO₄)₂ (TCP),
- SrHPO₄ and/or Sr₃(PO₄)₂,
- optionally SrCO₃ in addition.

10. The preparation according to claim 9, wherein the powder mixture further contains NaH₂PO₄ and/or Na₂HPO₄, KH₂PO₄ and/or K₂HPO₄, or combinations of said Na- and K-salts of the orthophosphoric acid.

11. A strontium apatite cement preparation, comprising a powder mixture component and an aqueous mixing solution component, wherein the powder mixture component contains the following ingredients:
- Ca₃(PO₄)₂ (TCP),
- SrHPO₄ and/or Sr₃(PO₄)₂,
- optionally SrCO₃ in addition,
and wherein the mixing solution component contains an aqueous solution of an alkali salt or an ammonium salt of orthophosphoric acid,
wherein the preparation may contain magnesium ions only in unavoidable traces.

12. The strontium apatite cement preparation according to claim 11, wherein the mixing solution component contains an aqueous solution of (NH₄)₂HPO₄ or an aqueous solution of a primary and/or secondary Na- and/or K-salt of the orthophosphoric acid.

13. The strontium apatite cement preparation according to claim 11, wherein the mixing solution component contains an aqueous solution of primary salt NaH₂PO₄ and/or KH₂PO₄, an aqueous solution of secondary salt K₂HPO₄ and/or Na₂HPO₄, or an aqueous solution of a mixture of primary salt KH₂PO₄ and/or NaH₂PO₄ with secondary salt K₂HPO₄ and/or Na₂HPO₄.

14. The strontium apatite cement preparation according to claim 11, wherein the powder mixture additionally contains NaH₂PO₄ and/or Na₂HPO₄, KH₂PO₄ and/or K₂HPO₄, or combinations of said Na- and K-salts of the orthophosphoric acid.

15. The strontium apatite cement preparation according to one of claims 11 to 14, wherein SrHPO₄ and/or Sr₃(PO₄)₂ is present in the powder mixture component in an amount of more than 10 wt.-% to 60 wt.-%.

16. The strontium apatite cement preparation according to one of claims 11 to 15, wherein SrCO₃ is present in the powder mixture component in an amount of about 0.01 wt.-% to 10 wt.-%.

17. The preparation according to one of claims 1 to 16,
wherein the powder component and/or the aqueous liquid component additionally contains a pharmacologically and/or biologically active substance.

18. The preparation according to claim 17, wherein the pharmacologically and/or biologically active substance is an antibiotic, a cytostatic agent, an analgetic agent, a disinfectant, a growth factor, a protein and/or a biopolymer.

19. The preparation according to claim 18, wherein the pharmacologically and/or biologically active substance is one selected from the following group: gentamicin or tobramycin, clindamycin, vancomycin, a substance of the TGF-β-series and a substance of the BMPs series.

20. The preparation according to one of claims 1 to 19, wherein the powder component additionally contains a substance in the form of granular particles, which dissolves in the aqueous liquid component.

21. A use of a cement preparation according to one of claims 1 to 20 for the manufacture of an agent for medical purposes.

22. A use of a preparation according to one of claims 1 to 20 for the manufacture of a bone substitute, a bone filler, a bone cement or a bone adhesive, or a therapeutic agent for the treatment of osteoporosis.

23. A process for producing a strontium apatite cement, comprising the steps of:
- mixing a powder component and an aqueous component, as set forth in one of claims 1 - 19, and
- allowing the mixture to harden under the formation of strontium apatite as reaction product.

24. The process according to claim 23, wherein a paste formed after mixing is filled into a mold, whereby defined molded bodies are produced after hardening of the paste in accordance with the mold as a matrix.

25. The process according to claim 23, wherein an injectable liquid is formed after the mixing and before the hardening of the mixture.

26. Strontium apatite, obtainable by a process for producing a strontium apatite cement according to claim 23.

## Revendications

1. Préparation de ciment d'hydroxy-phosphate de strontium-calcium durcissable, comprenant :
- un mélange de poudre en des quantités molaires des composants que sont le calcium (Ca), le strontium (Sr) et l'ortho-phosphate (P) dans le mélange, dans les fourchettes de 1,00 < Ca/P ≤ 1,50 et 0 < Sr/P < 1,50
- un sel alcalin ou un sel d'ammonium de l'acide ortho-phosphorique,
et
- de l'eau et/ou une solution aqueuse,
la préparation pouvant contenir des ions magnésium, uniquement sous forme de traces inévitables.

2. Préparation selon la revendication 1, le sel alcalin de l'acide ortho-phosphorique étant un sel de sodium (Na-) et/ou de potassium (K-).

3. Préparation selon la revendication 1 ou 2, le sel alcalin de l'acide ortho-phosphorique étant sélectionné dans un groupe composé des composants suivants :
un sel de potassium primaire, un sel de potassium secondaire, de l'acide ortho-phosphorique et un mélange de ceux-ci,
un sel de sodium primaire, un sel de sodium secondaire de l'acide ortho-phosphorique et un mélange de ceux-ci,
et des combinaisons des sels de potassium et de sodium cités.

4. Préparation selon l'une des revendications précédentes, le sel alcalin ou d'ammonium de l'acide ortho-phosphorique se présentant sous la forme de solution aqueuse.

5. Préparation selon l'une des revendications précédentes, la solution aqueuse présentant une valeur de pH dans la fourchette de 7 à 12.

6. Préparation selon l'une des revendications précédentes, le strontium dans le mélange de poudre se présentant sous la forme de phosphate de strontium ou d'hydrogéno-phosphate de strontium ou un mélange de ceux-ci.

7. Préparation selon la revendication 6, le phosphate de strontium, l'hydrogéno-phosphate de strontium ou le mélange de ceux-ci dans le mélange de poudre se présentant en une quantité de plus de 10 % en poids à 60 % en poids.

8. Préparation selon la revendication 6, le mélange de poudre contenant en plus du carbonate de strontium.

9. Préparation selon l'une des revendications précédentes, le mélange de poudre contenant les composants suivants :
- Ca₃ (PO₄)₂ (TCP),
- SrHPO₄ et/ou Sr₃(PO₄)₂
- le cas échéant, en plus, du SrCO₃.

10. Préparation selon la revendication 9, le mélange de poudre contenant en plus du NaH₂PO₄ et/ou Na₂HPO₄, KH₂PO₄ et/ou K₂HPO₄ ou des combinaisons des selon de sodium et de potassium cités de l'acide ortho-phosphorique.

11. Préparation de ciment d'apatite de strontium, avec un composant formé d'un mélange de poudre et un autre formé d'une solution de mélange aqueuse,
le composant de mélange de poudre contenant les constituants suivants :
- Ca₃(PO₄)₂ (TCP),
- SrHPO₄ et/ou Sr₃(PO₄)_{2,}
- le cas échéant, en plus, du SrCO₃,
et où le composant de solution en mélange, contenant une solution aqueuse, d'un sel alcalin ou d'un sel d'ammonium de l'acide ortho-phosphorique, la préparation pouvant contenir des ions magnésium, uniquement sous forme de traces inévitables.

12. Préparation de ciment d'apatite de strontium selon la revendication 11, le composant de solution en mélange contenant une solution aqueuse de (NH₄)₂HPO₄ ou une, solution aqueuse d'un sel de Na et/ou de K primaire et/ou secondaire de l'acide ortho-phosphorique.

13. Préparation de ciment d'apatite de strontium selon la revendication 11, le composant de solution en mélange contenant une solution aqueuse de sel primaire NaH₂PO₄ et/ou KH₂PO₄, une solution aqueuse de sel secondaire K₂HPO₄ et/ou Na₂HPO₄, ou une solution aqueuse d'un mélange composé de sel primaire KH₂PO₄ et/ou NaH₂PO₄ avec un sel secondaire K₂HPO₄ et/ou Na₂HPO₄.

14. Préparation de ciment d'apatite de strontium selon la revendication 11, le mélange de poudre contenant en plus du NaH₂PO₄ et/ou du Na₂HPO₄, KH₂PO₄ et/ou K₂HPO₄ ou des combinaisons de sels de Na et de K cités de l'acide ortho-phosphorique.

15. Préparation de ciment d'apatite de strontium selon l'une des revendications 11 à 14, dans le composant de mélange en poudre, le SrHPO₄ et/ou Sr₃(PO₄)₂ se présentant en une quantité de plus de 10 % en poids jusqu'à 60 % en poids.

16. Préparation de ciment d'apatite de strontium selon l'une des revendications 11 à 15, où dans le composant de mélange en poudre, SrCO₃ se présente en une quantité de 0,01 à 10 % en poids.

17. Préparation selon l'une des revendications 1 à 16, où le composant en poudre et/ou le composant liquide aqueux contenant en plus une substance pharmacologiquement et/ou biologiquement active.

18. Préparation selon la revendication 17, la substance pharmacologiquement et/ou biologiquement active étant un antibiotique, un cytostatique, un analgésique, un produit désinfectant, un facteur de croissance, une protéine et/ou un biopolymère.

19. Préparation selon la revendication 18, la substance pharmacologiquement et/ou biologiquement active étant une substance sélectionnée dans le groupe suivant : gentamicine ou tobramycine, clindamycine, vancomycine, une substance de la série TGF-β et une substance de la série des BPMs.

20. Préparation selon l'une des revendications 1 à 19, le composant en poudre contenant en plus une substance se présentant sous la forme de particules granulaires se dissolvant dans le composant liquide aqueux.

21. Utilisation d'une préparation de ciment selon l'une des revendications 1 à 20, pour la préparation d'un produit à des fins médicales.

22. Utilisation d'une préparation selon l'une des revendications 1 à 20, pour la préparation d'une substance de substitution osseuse, d'une substance de remplissage osseux, un ciment pour os ou une colle à os ou un produit thérapeutique pour le traitement de l'ostéoporose.

23. Procédé de préparation d'un ciment d'apatite de strontium, présentant les étampes :
- mélange d'un composant en poudre à un composant aqueux, tel qu'indiqué à l'une des revendications 1 à 19, et
- laisser durcir le mélange en formant de l'apatite de strontium en tant que produit final de réaction.

24. Procédé selon la revendication 23, une pâte formée après le mélange étant introduite dans un moule, faisant que, après durcissement de la pâte, un corps de forme défini, suivant le moule servant de matrice, est fabriqué.

25. Procédé selon la revendication 23, sachant que, après mélange et avant durcissement du mélange, un liquide injectable est formé.

26. Apatite de strontium obtenu par un procédé de préparation d'un ciment d'apatite de strontium selon la revendication 23.
